# EUROPÄISCHE PATENTSCHRIFT

(11) **EP 2 404 590 B1**
(45) Veröffentlichungstag und Bekanntmachung des Hinweises auf die Patenterteilung: **12.06.2019**
(21) Anmeldenummer: 10007066.3
(22) Anmeldetag: 08.07.2010
(51) Int. Cl.: A61K 6/083, C04B 35/634

(54) **Lichthärtende Keramikschlicker für die stereolithographische Herstellung von hochfesten Keramiken**
Light hardening ceramic dross for stereolithographic production of highly stable ceramics
Barbotine en céramique durcissant à la lumière pour la fabrication stéréo-lithographique de céramiques à haute résistance

(43) Veröffentlichungstag der Anmeldung: 11.01.2012
(73) Patentinhaber: Ivoclar Vivadent AG, 9494 Schaan (LI)
(72) Erfinder: Fischer, Urs Karl, 9320 Arbon (CH); Moszner, Norbert, 9495 Triesen (LI); Rheinberger, Volker, 9490 Vaduz (LI); Wachter, Wolfgang, 9494 Schaan (LI); Homa, Johannes, 1130 Wien (AT); Längle, Werner, 6820 Frastanz (AT)
(74) Vertreter: Uexküll & Stolberg

(56) Entgegenhaltungen:
- EP-A1- 2 151 214
- EP-A2- 2 077 103
- WO-A1-99/03444
- US-A1- 2008 206 715

## Beschreibung

Die vorliegende Erfindung betrifft lichthärtende Keramikschlicker für die stereolithographische Herstellung von hochfesten Keramiken, wie z.B. Keramikformteilen, dentalen Inlays, Onlays, Veneers, Kronen, Brücken und Gerüsten.

Unter dem Begriff "Rapid Prototyping" (RP), werden generative Fertigungsverfahren zusammengefasst, bei denen aus computergestützten Konstruktionsdaten (CAD-Daten) 3-dimensionale Modelle oder Bauteile hergestellt werden (A. Gebhardt, Vision of Rapid Prototyping, Ber. DGK 83 (2006) 7-12). Dabei handelt es sich um Verfahren, wie z.B. Stereolithographie (SL), selektives Lasersintern (SLS), 3D-Printing, Fused Deposition Modelling (FDM), Ink-Jet-Printing (IJP), 3D-Plotting, Multi-Jet Modelling (MJM), Solid Freeform Fabrication (SFF), Laminated Object Manufacturing (LOM), Laser Powder Forming (LPF) und Direct Ceramic Jet Printing (DCJP), mit denen sich Modelle, Bau- oder Formteile auch in Kleinserie kostengünstig herstellen lassen (A. Gebhardt, Generative Fertigungsverfahren, 3. Aufl., Carl Hanser Verlag, München 2007, 77 ff.). Bei der Stereolithographie handelt es sich um RP-Verfahren (A. Beil, Fertigung von Mikro-Bauteilen mittels Stereolithographie, Düsseldorf 2002, VDI-Verlag 3 ff.), bei denen ein Formteil auf der Basis von CAD-Daten schichtweise aus einem flüssigen und härtbaren Monomerharz aufgebaut wird.

Stereolithographische Verfahren zur Herstellung von dentalen Formkörpern, wie Inlays, Kronen oder Brücken, sind vor allem bei keramischen Werkstoffen von großem Vorteil, da damit eine deutliche Vereinfachung der im zahntechnischen Labor mit großem manuellen Aufwand betriebenen Abform- und Gießprozesse bzw. der Fräs- und Schleifoperationen möglich ist und gleichzeitig der bei nicht-generativen Verfahren auftretende große Materialverlust vermieden werden kann. Da heutzutage eine vollständige digitale Prozesskette etabliert ist, lassen sich die klassischen Verfahrensschritte zur Herstellung von z.B. mehrgliedrigen Brückengerüsten (Ausrichten im Artikulator, Wachsmodulation, Einbetten und Guss) durch die Digitalisierung des Modells, virtuelle Konstruktion des dentalen Formkörpers und dessen generative stereolithographische Fertigung ersetzen.

Bei der stereolithographischen Herstellung von keramischen Formteilen wird zunächst ein keramischer Grünling durch schichtweise Strahlungshärtung eines fließfähigen keramischen Schlickers hergestellt, der dann nach der Entbinderung zu einem dichten keramischen Formkörper gesintert wird. Der Grünling wird auch Grünkörper genannt. Als Entbinderung wird das Austreiben des Bindemittels bezeichnet. Hierbei wird das verwendete Bindemittel üblicherweise durch Erhitzen des Grünlings auf eine Temperatur von ca. 90 °C bis 600 °C entfernt. Wesentlich ist, dass die Bildung von Rissen und Deformationen weitestgehend vermieden wird. Durch die Entbinderung wird aus dem Grünling der sogenannte Weißling.

Beim Entbindern finden rein thermische wie auch thermochemische Prozesse statt. Üblicherweise werden beim Verpressen von keramischen Pulvern als Binder Mischungen von Wasser, Lösungsmitteln, Polymeren, Wachsen oder Ölen eingesetzt. Als Polymere finden meist Polypropylen, Polyethylen, Polyvinylacetat, Polyvinylalkohol, Methylcellulose, Polyvinylpyrrolidon, Polystyrol oder Polyethylmethacrylat Anwendung (vgl. R. Moreno, Amer. Cer. Soc. Bull. 71 (1992) 1647-1657). Hierbei handelt es sich um lineare Polymere, die durch Depolymerisation oder Kettenspaltung bei höherer Temperatur mehr oder weniger leicht zu flüchtigen Komponenten abgebaut werden.

Die Sinterung des Weißlings erfolgt im Sinterofen beim Hochtemperaturbrand. Dabei kommt es zur Verdichtung und Verfestigung des fein verteilten Keramikpulvers durch Temperatureinwirkung unterhalb der Schmelztemperatur der Hauptkomponenten, wodurch das poröse Bauteil kleiner wird und dessen Festigkeit zunimmt.

Die EP 1 021 997 A2 beschreibt die Anwendung des Lasersinterverfahrens für die Herstellung von Zahnrestaurationen. Hierbei werden Metallpulver schichtweise unter Einsatz eines Lasers gesintert.

Die DE 101 14 290 A1 betrifft die Herstellung von dentalen Formteilen durch 3D-Plotting unter Verwendung von schmelzbaren, kondensierbaren, thermisch oder mit UV- oder sichtbarem Licht härtbaren, ungefüllten oder gefüllten Materialien. Zur Herstellung von Grünkörpern werden anorganische Pasten vorgeschlagen, die aus Glas-, Glaskeramik- oder Keramikpulver bestehen, das mit Lösungsmittel, Binder und Plastifikator in eine formbare Paste überführt wird. Die verwendeten Pulver sind nicht oberflächenmodifiziert.

Die WO 97/29901 beschreibt ein Verfahren und ein Gerät zur Herstellung 3-dimensionaler Teile aus einem flüssigen, härtbaren Medium. Dabei wird das Teil schichtenweise aufgebaut, indem jede einzelne Schicht mit einem Laser abgefahren und dabei ausgehärtet wird. Danach wird mittels eines Abstreifers die nächste Schicht des härtbaren Materials aufgetragen und anschließend ebenso gehärtet.

Ein stereolithographisches Verfahren zur Herstellung von Dentalimplantaten ist aus der WO 95/28688 bekannt.

Die US 5,496,682 offenbart lichthärtbare Zusammensetzungen für die Herstellung dreidimensionaler Körper durch Stereolithographie, die 40 bis 70 Vol.-% Keramik- oder Metallpartikel, 10 bis 35 Gew.-% Monomer, 1 bis 10 Gew.-% Photoinitiator, 1 bis 10 Gew.-% Dispergiermittel und vorzugsweise auch Lösungsmittel, Weichmacher und Kopplungsmittel enthalten.

Die US 6,117,612 beschreibt Harze für die stereolithographische Herstellung von gesinterten Keramik- oder Metallteilen. Die Harze haben eine Viskosität von weniger als 3000 mPa·s. Zur ihrer Herstellung werden Monomere mit geringer Viskosität eingesetzt, vorzugsweise in wässriger Lösung. Durch die Verwendung von Dispersionsmitteln soll ein hoher Feststoffgehalt bei geringer Viskosität erzielt werden.

Die DE 10 2005 058 116 A1 offenbart Suspensionen zur stereolithographischen Herstellung von keramischen Implantaten auf die in der US 6,117,612 beschriebene Weise, die keine Verdünnungsmittel wie Wasser oder organische Lösungsmittel enthalten, da diese durch lokales Verdampfen beim Energieeintrag die Viskosität erhöhen sollen. Die Viskosität der Suspension wird durch Variation der Konzentration eines Dispergators auf weniger als 20 Pa·s eingestellt. Als Dispergator werden Alkylammoniumsalze von Copolymeren mit sauren Gruppen eingesetzt, wobei diese auch auf die Partikel des keramischen Pulvers geschichtet werden können.

In der US 2005/0090575 A1 werden Methoden und Zusammensetzungen für die stereolithographische Herstellung von keramischen Bauteilen beschrieben. Es wird angegeben, dass mit den aus US 5,496,682 bekannten flüssigen Materialien hergestellte Formteile weich sind und daher einen zusätzlichen Härtungsschritt erfordern, um Verformungen beim Brennen zu vermeiden, während aus pastenförmigen Materialien gewonnene Formkörper bei der Entbinderung innere Spannungen aufbauen, die beim Sintern zu Rissen führen. Zur Vermeidung dieser Probleme werden Weichmacher eingesetzt und die Menge des Keramikpulvers so gewählt, dass die Viskosität der Zusammensetzungen mindestens 10.000 Pa·s beträgt.

In den DE 199 38 463 A1 und DE 199 50 284 A1 werden mit sichtbarem Licht härtbare Zusammensetzungen und deren Verwendung zur Herstellung von Dentalrestaurationen aus Kunststoffmaterialen mit RP-Verfahren beschrieben.

Die EP 2 151 214 A1 offenbart lichthärtende Schlicker für die stereolithografische Herstellung von Keramikformteilen, die ein polyreaktives Bindemittel, einen Photoinitiator und oberflächenmodifizierte Keramik- oder Glaskeramikpartikel enthalten. Als Bindemittel können radikalisch und kationisch polymerisierbare Harze, Monomere für die ringöffnende Metathesepolymerisation, Thiol-En-Harze und Michael-Reaktionsharze verwendet werden.

Die EP 2 077 103 A2 offenbart lichthärtende Dentalzemente, die mindestens ein acides polymerisierbares Monomer, mindestens ein nicht-acides Monomer, einen Photoinitiator und Füllstoff enthalten Als Füllstoffe werden anorganische Metallverbindungen und Gläser genannt.

Die US 2008/0206715 A1 offenbart dentale Adhäsive, die eine polymerisierbare Komponente, einen Polymerisationsinitiator und mindestens eine Komponente enthalten, die eine pHabhängige Farbänderung zeigt. Die Zusammensetzungen können außerdem nicht-reaktive Füllstoffe enthalten.

Die WO 99/03444 A1 betrifft dentale Zemente, die einen Füllstoff, ein polymerisierbares Harz und ein polymeres Modifizierungsmittel zur Verbesserung der Handhabung enthalten. Das Modifizierungsmittel kann acide Gruppen aufweisen. Ein bevorzugtes Modifizierungsmittel ist methacrylatmodifiziertes Polytetramethylenoxid.

Für die Herstellung von hochfesten Keramiken muss die Prozesskette so durchgeführt werden, dass es bei der Herstellung des Grünkörpers, Braunlings und Weißlings nicht zur Bildung von Rissen, getrennten Schichten, Poren oder anderen Defekten oder Deformationen kommt. Diesbezüglich besitzen die Zusammensetzung und die Eigenschaften des strahlungshärtbaren Schlickers eine entscheidende Bedeutung. So ist insbesondere für eine hohe Dichte und Endfestigkeit sowie gute Passgenauigkeit des keramischen Formteils ein möglichst hoher Volumenanteil der keramischen Partikel im Schlicker notwendig. Weiterhin ist eine gut eingestellte Rheologie des Schlickers eine grundlegende Voraussetzung für einen schnellen und problemlosen stereolithographischen Aufbau eines defektarmen Grünkörpers, wobei aber die Viskosität und das Fliessverhalten u.a. von der Grösse und dem Gehalt der Keramikpartikel im Schlicker sowie von der Art und Menge zugesetzter Rheologieadditive abhängen.

Die Stabilität der stereolithographisch erzeugter Grünlinge kann durch die Verwendung vernetzender Monomere verbessert werden. Hierdurch kann die Härtungszeit, die erforderlich ist, um einen stabilen Festkörper zu erhalten, deutlich verkürzt werden. Gleichzeitig weist das sich ausbildende Polymernetzwerk im Vergleich zu linearen Polymeren aber auch eine deutlich erhöhte thermische Stabilität auf, was den Entbinderungsprozess nachteilig beeinflusst. Außerdem nimmt mit der Funktionalität der Monomeren ihr Polymerisationsschrumpf zu, was zu erheblichen Schrumpfungsspannungen führen kann. Als Folge davon werden auch bei optimierten Verarbeitungsprozessen häufig Grünkörper, Braunlinge und gesinterte Weißlinge erhalten, die Porositäten und Defekte aufweisen. Ein besonderes Problem bei der stereolithographischen Herstellung von Formkörpern liegt darin, dass die einzelnen Schichten ungenügend aneinander haften, was zu Schichtentrennung führen kann.

Die bekannten Schlicker sind hinsichtlich der oben genannten Anforderungen nicht zufriedenstellend. Der Erfindung liegt daher die Aufgabe zugrunde, lichthärtende Keramikschlicker zur stereolithographischen Herstellung von Keramikformteilen bereitzustellen, die eine gute stereolithographische Verarbeitbarkeit zeigen, ausreichend feste und formstabile Grünkörper ergeben und nach der Entbinderung und Sinterung zu defektfreien, hochfesten Keramiken führen. Insbesondere soll eine Schichtentrennung verhindert werden.

Erfindungsgemäß wird diese Aufgabe durch Schlicker auf der Basis von radikalisch polymerisierbarem Bindemittel, Polymerisationsinitiator und Füllstoff gelöst, die die folgenden Komponenten enthalten:
**A)** 1 bis 30 Gew.-% mindestens eines aciden Monomers der allgemeinen Formel I 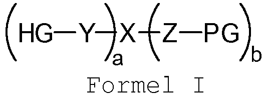 in der
   - X: entfällt oder ein (a+b)-wertiger, substituierter oder unsubstituierter, geradkettiger aliphatischer Rest mit mindestens 2, vorzugsweise 3 bis 15 Kohlenstoffatomen, ein verzweigter aliphatischer Rest mit mindestens 3, vorzugsweise 3 bis 15 Kohlenstoffatomen, ein cycloaliphatischer, bi- oder tricylischer oder aromatischer Rest mit 6 bis 20, vorzugsweise 6 bis 15 Kohlenstoffatomen oder eine Kombination dieser Reste ist, vorzugsweise ein aromatischer oder cycloaliphatischer C₆-Rest und insbesondere ein geradkettiger oder verzweigter aliphatischer C₃-C₁₅-Rest, besonders bevorzugt C₃-C₈-Rest ist, wobei diese Gruppen 1 oder mehrere Ethergruppen enthalten können;
   - PG: eine radikalisch polymerisierbare Gruppe, vorzugsweise eine Vinyl- (CH₂=CH-), Allyl- (CH₂=CH-CH₂-) , Allylether- (CH₂=CH-CH₂-O-), Styryl (CH₂=CH-Phenyl-), (Meth)acrylat- (CH₂=C(-H/-CH₃)-CO-O-) oder (Meth)-acrylamid-Gruppe (CH₂=C(-H/-CH₃)-CO-NH- oder CH₂=C(-H/-CH₃) -CO-NR- mit R= C₁-C₄-Alkyl) , besonders bevorzugt eine (Meth)acrylat- oder (Meth)acrylamid-Gruppe und ganz besonders bevorzugt eine Acrylat- (CH₂=CH-CO-O-) oder Acrylamid-Gruppe (CH₂=CH-CO-NH-) ist;
   - HG: -COOH ist;
   - Y: entfällt oder eine zweiwertige, geradkettige aliphatische Gruppe mit 2 bis 40 Kohlenstoffatomen, eine verzweigte aliphatische Gruppe mit 3 bis 40 Kohlenstoffatomen oder eine cycloaliphatische oder aromatische Gruppe mit 5 bis 40 Kohlenstoffatomen ist, wobei Y eine oder mehrere Ether-, Thioether-, Amid-und/oder Estergruppen enthalten kann, vorzugsweise ein geradkettiger aliphatischer Rest mit 2 bis 10 Kohlenstoffatomen, ein verzweigter aliphatischer Rest mit 3 bis 12 Kohlenstoffatomen, ein cycloaliphatischer, bi- oder tricylischer oder aromatischer Rest mit 6 bis 15 Kohlenstoffatomen oder eine Kombination dieser Reste ist;
   - Z: entfällt oder eine zweiwertige, geradkettige aliphatische Gruppe mit 2 bis 40 Kohlenstoffatomen oder eine verzweigte aliphatische Gruppe mit 3 bis 40 Kohlenstoffatomen ist, wobei Z eine oder mehrere Ether-, Thioether-, Amid- und/oder Estergruppen enthalten kann, vorzugsweise ein geradkettiger aliphatischer Rest mit 2 bis 10 Kohlenstoffatomen, ein verzweigter aliphatischer Rest mit 3 bis 12 Kohlenstoffatomen, ein cycloaliphatischer, bi- oder tricylischer oder aromatischer Rest mit 6 bis 15 Kohlenstoffatomen, eine Kombination dieser Reste oder ein Oligoethylenglycol- (-(CH₂-CH₂-O)ₙ-, n = 1-5) oder ein Oligopropylenglycolrest (-(CH(CH₃)-CH₂-O)ₙ-, n = 1-5) ist;
   - a: eine ganze Zahl von 1 bis 5, vorzugsweise 1, 2 oder 3 ist; und
   - b: 1 oder 2, vorzugsweise 1 ist;
**A2)** 0 bis 50 Gew.-% mindestens eines nicht aciden radikalisch polymerisierbaren Monomers;
**B)** 0,001 bis 2,0 Gew.-% Photoinitiator; und
**C)** 30 bis 90 Gew.-% Keramik- und/oder Glaskeramikpartikel;
jeweils bezogen auf die Gesamtmasse des Schlickers,
wobei der Schlicker als Komponente **(C)** Glaskeramikpartikel oder Keramikpartikel auf der Basis von reinem ZrO₂, reinem Al₂O₃, reinem ZrO₂-Al₂O₃, ZrO₂, das mit HfO₂, CaO, Y₂O₃, CeO₂ und/oder MgO stabilisiert ist, ZrO₂-Al₂O₃, das mit HfO₂, CaO, Y₂O₃, CeO₂ und/oder MgO stabilisiert ist, oder Nicht-OxidKeramiken enthält.

Die in Y und/oder Z gegebenenfalls vorhandenen Estergruppen haben vorzugsweise 1 bis 4 Kohlenstoffatome. Der Amidstickstoff der (Meth)acrylamid-Gruppen kann durch Alkyl-, vorzugsweise C₁-C₃-Alkyl substituiert sein.

Die erfindungsgemäßen Schlicker enthalten als radikalisch polymerisierbares Bindemittel mindestens ein acides Monomer gemäß der allgemeinen Formel (I) (Komponente **A**), vorzugsweise eine Mischung aus mindestens einem aciden Monomer und mindestens einem nicht aciden Monomer (Komponente **A2**). Das acide Monomer wird im Folgenden auch als Haftmonomer und das nicht acide Monomer als Comonomer bezeichnet.

Es wurde überraschender Weise gefunden, dass die erfindungsgemäßen Schlicker nur einen geringen Polymerisationsschrumpf aufweisen und so die Herstellung von dimensionsstabilen, flexiblen Grünkörpern mit guter Formstabilität und geringer Deformationsspannung führen. Die Schlicker ergeben defektfreie Grünkörper, die sich durch eine hervorragende Haftung zwischen den einzelnen stereolithographisch erzeugten Schichten auszeichnen. Nach dem Entbindern und Sintern der Grünkörper werden hochfeste Keramik- bzw. Glaskeramikformteile erhalten, die sich insbesondere für dentale Zwecke eignen. Weiterhin hat sich sehr überraschend gezeigt, dass auch monofunktionelle Haftmonomere der Formel (I) eine außergewöhnlich hohe radikalische Photopolymerisationsaktivität aufweisen. Unter monofunktionellen Monomeren werden Monomere mit einer radikalisch polymerisierbaren Gruppe und unter multifunktionellen Monomeren Monomere mit zwei oder mehr radikalisch polymerisierbaren Gruppen verstanden. Monomere mit zwei oder mehr radikalisch polymerisierbaren Gruppen wirken vernetzend und werden daher auch als Vernetzermonomere bezeichnet.

COOH-Gruppen-haltige Monomere sind z.B. durch stöchiometrische Addition von Hydroxyalkyl(meth)acrylaten oder N-(Hydroxyalkyl)-(meth)acrylamiden mit aliphatischen, cycloaliphatischen, bi- oder tricyclischen oder aromatischen Di-, Tri- oder Tetracarbonsäureanhydriden zugänglich. Bevorzugt sind Anhydride von Dicarbonsäuren. Dafür geeignete Hydroxyalkyl(meth)acrylate oder N-(Hydroxyalkyl)-(meth)acrylamiden sind 2-Hydroxyethyl(meth)acrylat, 2-Hydroxypropyl(meth)-acrylat, 4-Hydroxybutyl(meth)acrylat, 10-Hydroxydecyl(meth)-acrylat, OH-terminierte Poly(ethylenglycol)- oder Poly-(propylenglycol)-mono(meth)acrylat, α-Hydroxymethylacrylsäureethylester, N-(2-Hydroxyethyl)(meth)acrylamid, N-Methyl-N-(2-hydroxyethyl)-acrylamid, N-(5-hydroxypentyl)(meth)acrylamid oder N-(10-hydroxydecyl(meth)acrylamid. Bevorzugte Dicarbonsäureanhydride sind Bernsteinsäure-, Maleinsäure-, Fumarsäure- oder Glutarsäureanhydrid, Cyclohexan-1,2-dicarborsäure-anhydrid, 1,2-Cyclopropandicarbonsäureanhydrid, Norbornen-2,3-dicarbonsäureanhydrid, das Diels-Alder-Addukt aus Maleinsäureanhydrid und 1,3-Cyclopentadien oder Phthalsäureanhydrid. Weiterhin lassen sich COOH-haltige Monomere durch ringöffnende Addition von di- oder höherfunktionalisierten Carbonsäuren, wie z.B. Oxal-, Malon-, Berstein-, Glutar-, Adipin-, Sebacin-, Pimelin-, Kork-, Malein-, Terephthal-, Isophthal-, Phthal-, Cyclohexandicarbon-, Gallus-, Wein-, oder Citronensäure an z.B. Glycidyl-(meth)acrylat oder 4-Vinyl-1-cyclohexen-1,2-epoxid synthetisieren oder sind als Halbester durch Veresterung der oben genannten Hydroxyalkyl(meth)acrylate bzw. N-(Hydroxyalkyl)-(meth)acrylamide mit den aufgeführten Dicarbonsäuren zugänglich.

Bevorzugte COOH-gruppenhaltige Monomere (HG = COOH) sind die Umsetzungsprodukte von Hydroxyalkylacrylaten, wie Hydroxyethyl- oder Hydroxypropylacrylat, mit aliphatischen, cycloaliphatischen oder aromatischen Carbonsäureanhydriden, insbesondere mit Bernsteinsäure-, Glutarsäure-, Cyclohexan-1,2-dicarbonsäure- oder Phthalsäureanhydrid.

Besonders bevorzugt sind Monomere mit COOH-Gruppen (HG = COOH), die als polymerisationsfähige Gruppe(n) PG Vinyl-, Allyl-, Allylether, Styryl, (Meth)acrylat- oder (Meth)acrylamid-Gruppen tragen, insbesondere (Meth)acrylat- oder (Meth)acrylamid-Reste, wobei Acrylat- oder Acrylamid-Reste ganz besonders bevorzugt sind.

Besonders bevorzugt sind Monomere gemäß der Formel (I) in der die Variablen unabhängig voneinander die folgenden Bedeutungen haben:
- X: entfällt oder ein (a+b)-wertiger, unsubstituierter, verzweigter oder geradkettiger aliphatischer Rest mit 3 bis 15, vorzugsweise 3 bis 8 Kohlenstoffatomen, der 1 oder 2 Ethergruppen enthalten kann;
- PG: eine Acrylat- oder Acrylamid-Gruppe,
- HG: -COOH,
- Y: eine zweiwertige, geradkettige aliphatische Gruppe mit 2 bis 8 Kohlenstoffatomen oder eine verzweigte aliphatische Gruppe mit 3 bis 8 Kohlenstoffatomen, wobei diese Gruppe 1 Estergruppe enthalten kann, und
- Z: eine zweiwertige, geradkettige aliphatische Gruppe mit 2 bis 10 Kohlenstoffatomen oder eine verzweigte aliphatische Gruppe mit 3 bis 10 Kohlenstoffatomen, wobei diese Gruppen 1 oder mehrere, vorzugsweise 0 bis 3 Ethergruppen enthalten kann,
- a: 1, 2 oder 3, und
- b: 1.

Besonders bevorzugt sind erfindungsgemäß solche Monomere der Formel (I), in denen alle Variablen eine der bevorzugten oder besonders bevorzugten Bedeutungen haben.

Das radikalisch polymerisierbare Bindemittel enthält vorzugsweise mindestens ein nicht acides Monomer **A2.** In den radikalisch polymerisierbaren Harzen lassen sich als nicht acide Comonomere insbesondere mono- oder multifunktionelle (Meth)acrylate, (Meth)acrylamide, Vinylmonomere oder deren Mischungen einsetzen. Beispiele für (Meth)acrylate sind Ethyl-, Hydroxyethyl-, Hydroxypropyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), Additionsprodukte aus 2-Hydroxyalkyl(meth)acrylat und Diisocyanaten, wie z.B. Hexamethylendiisocyanat oder 2,2,4-Trimethylhexamethylendiisocyanat, Di-, Tri- oder Tetraethylenglycoldi(meth)acrylat, (meth)acrylat-terminierte Poly(ethylenglycol)e bzw. Poly-(propylenglycol)e, Trimethylolpropantri(meth)acrylat, Pentaerythrittetra(meth)acrylat, sowie Glycerindi- und -tri(meth)acrylat, 1,4-Butandioldi(meth)acrylat, 1,10-Decandioldi(meth)acrylat oder 1,12-Dodecandioldi(meth)acrylat.

Bevorzugte Beispiele für (Meth)acrylamide sind N-Ethylacrylamid, N,N-Dimethacrylamid, N-(2-Hydroxyethyl)acrylamid, N-Methyl-N-(2-hydroxyethyl)acrylamid, N-Ethylmethacrylamid oder N-(2-Hydroxyethyl)methacrylamid, N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin. Geeignete Vinylmonomere sind N-Vinylpyrrolidon oder Vinylacetat.

Die erfindungsgemäßen Schlicker enthalten vorzugsweise weniger als 40 Gew.-% und besonders bevorzugt weniger als 20 Gew.-% an Vernetzermonomeren bezogen auf die Gesamtmasse an radikalisch polymerisierbaren Komponenten.

Als Komponenten **B** können die erfindungsgemäßen Keramikschlicker bzw. Glaskeramikschlicker die bekannten radikalischen Photoinitiatoren (vgl. J.P. Fouassier, J.F. Rabek (Hrsg.), Radiation Curing in Polymer Science and Technology, Vol. II, Elsevier Applied Science, London und New York 1993) für den sichtbaren Bereich enthalten, wie z.B. Acyl- der Bisacylphosphinoxide, Titanocen-Photoinitiatoren, wie z.B. Bis(η⁵-cyclopentadienyl)bis[2,6-difluor-3-(1H-pyrr-1-yl)phenyl-titanium oder Bis (η⁵-methylcyclopentadienyl)bis[2, 3, 4, 5, 6-pentafluorphenyl)-titanium, aber vor allem α-Diketone wie 9,10-Phenanthrenchinon oder Diacetyl, Furil, oder 4,4'-Dialkoxybenzil und Campherchinon. Weiterhin geeignete Photoinitiatoren für den sichtbaren Bereich sind Kombinationen von photoreduzierbaren Farbstoffen, wie Acridin-, Xanthen-, Azin- oder Polymethin-Fabstoffe mit solchen Beschleunigern, wie z.B. Phosphine, Sulfinate, Enolate oder geeignete Borate (vgl. J.V. Crivello, K. Dietliker, Photoinitiators for Free Radical, Cationic & Anionic Photopolymerization, 2nd Ed., In: Surface Coating Technology, Editor: G. Bradley, Vol. III, Wiley&Sons, Chichester etc. 1998, 239 ff.). Besonders geeignet sind aminfreie Norrish-Typ-I-Photoinitiatoren, die oberhalb von 400 nm absorbieren, vor allem Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermnium sowie Bisacylphosphinoxide, die alle auch ohne Aminbeschleuniger zu einer hohen Polymerisationsgeschwindigkeit führen. Dabei lassen sich auch Mischungen der verschiedenen Photoinitiatoren einsetzen, wie z.B. Mischungen aus Dibenzoyldiethylgermanium in Kombination mit einem Bisacylphosphinoxid.

Als Beschleuniger für α-Diketone können Reduktionsmittel, wie z.B. 4-(Dimethylamino)-benzoesäureester, N,N-Dimethylaminoethylmethacrylat, N,N-Dimethyl-sym.-xylidin oder Triethanolamin eingesetzt werden. Weitere Beschleuniger sind Ascorbinsäure, Barbiturate oder Sulfinsäuren, die vor allem in Kombination mit geeigneten Peroxiden vorteilhaft sind. Außerdem können auch bekannte kationische Photoinitiatoren, wie z.B. Diaryliodonium- oder Triarylsulfoniumsalze, als Beschleuniger verwendet werden.

Photoinitiatoren werden vorzugsweise in eine Menge von 0,001-2,0 Gew.-%, besonders bevorzugt 0,01 - 1 Gew.-%, und Beschleuniger in einer Menge von 0 bis 2,0 Gew.-%, besonders bevorzugt 0,01 bis 1 Gew.-% verwendet.

Als Komponente C enthalten die erfindungsgemäßen Schlicker Keramik- und/oder Glaskeramikpartikel.

Unter Keramiken werden anorganische Werkstoffe verstanden, die eine kristalline Struktur aufweisen und meist aus entsprechenden Pulvern hergestellt werden. Vorzugsweise erfolgt die Herstellung der Keramik durch Sintern (Sinterkeramik). Oxidkeramiken werden vorzugsweise durch Sintern von Metalloxidpulvern wie z.B. ZrO₂ oder Al₂O₃ erhalten. Darüber hinaus können Oxidkeramiken auch eine oder mehrere Glasphasen enthalten. Bei Glaskeramiken handelt es sich um Werkstoffe, die meist aus amorphen Gläsern, insbesondere Silikatgläsern, durch gesteuerte Kristallisation hergestellt werden und bei denen eine Glasphase und eine oder mehrere Kristallphasen im Festkörper nebeneinander vorliegen. Bei sinterbaren Glaskeramiken kann sowohl von Glaspulvern als auch von Glaskeramikpulvern ausgegangen werden.

Bevorzugt sind sinterbare Glaskeramikpartikel auf Basis Leucit- oder Lithiumdisilikat-verstärkten Gläsern. Geeignete Keramikpartikel sind Keramikpartikel auf der Basis von reinem ZrO₂ oder reinem Al₂O₃, Partikel auf der Basis von mit HfO₂, CaO, Y₂O₃, CeO₂und/oder MgO stabilisiertem ZrO₂, Partikel auf der Basis von reinem ZrO₂-Al₂O₃ oder mit HfO₂, CaO, Y₂O₃, CeO₂ und/oder MgO stabilisiertem ZrO₂-Al₂O₃.

Der Begriff "rein" ist im Sinne von "chemisch rein" zu verstehen, d.h. eine ZrO₂- bzw. Al₂O₃-Keramik ist nur aus ZrO₂ bzw. Al₂O₃ aufgebaut. Stabilisierte Keramiken enthalten neben dem Basisoxid wie ZrO₂ oder Al₂O₃ ein Stabilisisierungsmittel, das vorzugsweise aus HfO₂, CaO, Y₂O₃, CeO₂, MgO und Mischungen davon ausgewählt ist. Das Stablilisierungsmittel wird vorzugsweise in einer Menge von 3 bis 5 Gew.-% eingesetzt, bezogen auf die Masse der stabilisierten Keramik. Hochfeste ZrO₂-Keramiken enthalten zur Stabilisierung der tetragonalen Kristallstruktur vorzugsweise 3 bis 5 Gew.-% Y₂O₃ (Yttriumoxid) . Diese ZrO₂-Keramik wird als Y-TZP (Yttrium Stabilized Tetragonal Zirconium Dioxide Polycrystals) bezeichnet. Keramikpartikel, die nur Basisoxid und Stabilisierungsmittel enthalten, sind besonders bevorzugt.

Die oben genannten Keramik- und Glaskeramikpartikel eignen sich besonders für dentale Anwendungen.

Die Partikelgröße der Komponente C liegt vorzugsweise im Bereich von 20 nm bis 50 µm. Sie ist abhängig von der eingesetzten Keramik. Bei Al₂O₃ liegt die Größe der als Komponente C verwendeten Partikel vorzugsweise im Bereich von 20 bis 5.000 nm, besonders bevorzugt zwischen 75 und 200 nm; bei Glaskeramik im Bereich von 100 nm und 50 µm, ganz bevorzugt zwischen 0,1 und 10 µm; bei Y-TZP Zirkondioxid im Bereich von 20 und 5000 nm, ganz bevorzugt zwischen 50 und 3.500 nm. Die Partikelgröße wird dabei vorzugsweise so gewählt, dass sedimentationsstabile Schlicker erhalten werden. Bei den Partikelgrößen handelt es sich um die absoluten Ober- und Untergrenzen.

Weiterhin lassen sich auch Keramik- oder Glaskeramikpartikel mit einer Partikelgröße im Bereich von 10-200 nm als Nano- der Organosole, d.h. als Dispersion der Nanopartikel in einem Lösungsmittel, einem geeigneten Monomer der Komponente **A** oder einer Mischungen daraus einsetzen.

Die Partikel werden gemäß einer bevorzugten Ausführungsform der Erfindung mit geeigneten Stoffen oberflächenmodifiziert. Zur Oberflächenmodifizierung werden vorzugsweise solche Verbindungen eingesetzt, die chemisch, d.h. durch ionische oder kovalente Bindungen an die Oberfläche der Keramik- oder Glaskeramikpartikel gebunden werden. Bevorzugt sind Verbindungen, die entweder Säuregruppen, vorzugsweise Carbonsäure-, Phosphonsäure-, Hydrogenphosphatgruppen oder saure Phosphorsäureestergruppen, oder Silylgruppen, vorzugsweise Alkoxysilylgruppen enthalten. Die Partikeloberfläche kann teilweise oder vorzugsweise vollständig mit dem Modifizierungsmittel bedeckt sein. Bei den erfindungsgemäß verwendeten Modifizierungsmitteln handelt es sich um monomere Verbindungen.

Dabei sind erfindungsgemäß solche Verbindungen besonders geeignet, die im Gegensatz zu den sogenannten Haftvermittlern oder Kopplungsreagenzien nur mit der Partikeloberfläche reagierende Gruppen enthalten, aber keine polyreaktiven Gruppen wie radikalisch polymerisierbaren Gruppen, z.B. (Meth)acryl-, (Meth)acrylamid-, Vinyl-, Vinylether- oder Epoxidgruppen, die mit der Harzmatrix (A) eine kovalente Bindung eingehen. Solche Verbindungen werden hierin als nicht polymerisierbare Oberflächenmodifikatoren bezeichnet. Diese Verbindungen haben den Vorteil, dass ein stabiler Verbund zwischen der Keramikpartikeloberfläche und der Polymermatrix im ausgehärteten Grünling nicht zustande kommt, was die vollständige Entfernung der Polymeranteile im Entbinderungsprozess vereinfacht.

Als nicht polymersisierbare Oberflächenmodifikatoren eigen sich insbesondere lineare oder verzweigte Carbonsäuren, wie z.B. Ameisensäure, Essigsäure, Propionsäure, Octansäure, Isobuttersäure, Isovaleriansäure, Pivalinsäure, saure Phosphorsäureester, wie z.B. Dimethyl-, Diethyl-, Dipropyl-, Dibutyl-, Dipentyl-, Dihexyl-, Dioctyl- oder Di(2-ethylhexyl)phosphat, oder Phosphonsäuren, z.B. wie Methyl-, Ethyl-, Propyl-, Butyl- Hexyl-, Octyl- oder Phenylphosphonsäure. Als nicht polymerisierbare Oberflächenmodifikatoren geeignete Silane sind beispielsweise Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan.

Gemäß einer bevorzugten Ausführungsform enthalten die erfindungsgemäßen Schlicker als Komponente **D** einen Inhibitor zur Verhinderung einer spontanen Polyreaktion als Stabilisator. Die Inhibitoren bzw. Stabilisatoren verbessern die Lagerstabilität der Schlicker und verhindern darüber hinaus eine unkontrollierte Polyreaktion in der stereolithographischen Wanne. Die Inhibitoren werden vorzugsweise in einer solchen Menge zugesetzt, dass die Schlicker über einen Zeitraum von ca. 2-3 Jahre lagerstabil sind. Besonders bevorzugt werden die Inhibitoren in einer Menge von 0,001 bis 1,0 Gew.-%, ganz besonders bevorzugt 0,001 bis 0,50 Gew.-% eingesetzt, jeweils bezogen auf die Gesamtmasse des Schlickers.

Für radikalische Reaktionsharze werden als sogenannte aerobe Inhibitoren Phenole, wie Hydrochinonmonomethylether (MEHQ) oder 2,6-Di-tert.-butyl-4-methyl-phenol (BHT), eingesetzt die nur in Gegenwart von Sauerstoff effektiv wirksam sind und meist in einem Konzentrationsbereich von 200-2000 ppm verwendet werden. Anaerobe Inhibitoren, wie z.B. Phenothiazin, 2,2,6,6-Tetramethyl-piperindin-1-oxyl-Radikal (TEMPO), Iod, Kupfer-(I)-iodid, wirken dagegen in geringsten Konzentrationen (10-50 ppm) auch bei Abwesenheit von Sauerstoff. Eine Polymerisation findet in der Regel erst dann statt, wenn diese Additive verbraucht sind. Dabei ist es auch häufig von Vorteil, eine Mischung von aeroben und anaeroben Inhibitoren einzusetzen.

Aerobe Inhibitoren werden vorzugsweise in einer Menge von 0,01 bis 0,50 Gew.-% und anaerobe Inhibitoren in einer Menge von 0,001 bis 0,02 Gew.-% eingesetzt, jeweils bezogen auf die Gesamtmasse des Schlickers. Bevorzugte Mischungen enthalten 0,02 bis 0,2 Gew.-%, insbesondere 0,01 bis 0,10 Gew.-% an aeroben Inhibitoren und/oder 0,001 bis 0,01 Gew.-% an anaeroben Inhibitoren, ebenfalls bezogen auf die Gesamtmasse des Schlickers.

Weiterhin lassen sich zur Vermeidung von Streueffekten oder zur Einstellung der Durchhärtungstiefe geeignete Farbstoffe, die vor allem im Wellenlängenbereich der verwendeten Photoinitiatoren bzw. Sensibilisatoren absorbieren, vor allem Azo- und Acridinfarbstoffe, zusetzen. Konkrete Beispiele hierfür sind 2,4-Dihydroxy-4-nitroazobenzol, Sudan Yellow 146, Sudan Orange 220, Sudan M Rot 380, Sudan gelb 177 flüssig. Schließlich können auch organische Weißpigmente bzw. Mattierungsmittel, wie z.B. auf Basis von PE-Wachsen, Polyamid 12 oder Polymethylharnstoffharzen zur Vermeidung von Streueffekten oder zur Einstellung der Durchhärtungstiefe verwendet werden. Durch die genannten Farbstoffe kann die Absorption des Schlickers kontrolliert beeinflusst (verringert) und dadurch die durch Streueffekte oder eine zu geringe Absorption des Schlickers verursachte Überstrahlung vermindert werden. Streuung und Überstrahlung können sich nachteilig auf die Formtreue des stereolithographisch hergestellten Bauteils auswirken.

Gemäß einer weiteren bevorzugten Ausführungsform der Erfindung enthalten die Schlicker als Komponente **E** einen sogenannten Entbinderungsbeschleuniger. Dieser wird vorzugsweise in einer Menge von 0 bis 20 Gew.-%, besonders bevorzugt 0,01 bis 10 Gew.-% eingesetzt, jeweils bezogen auf die Gesamtmasse des Schlickers. Unter Entbindungsbeschleunigern werden Stoffe verstanden, welche das Entfernen des Bindemittels während des Entbinderungsprozesses erleichtern.

Die Entbinderung des Grünlings kann durch geeignete, d.h. polyreaktionswirksame Stoffe im Polyreaktionsharz gefördert oder zielgerichtet beeinflusst werden. Dabei handelt es sich einerseits um Additive, die die Netzwerkbildung beeinflussen, wie vor allem kettenübertragungsaktive Stoffe, sog. Kettenregler, die zu einer Verringerung der Polymernetzwerkdichte und damit zu einer besseren thermischen Abbaubarkeit führen. Bekannte Kettenregler z.B. für die radikalische Polymerisation sind vor allem Mercaptane, wie z.B. Laurylmercaptan, und Disulfide. Disulfide, vor allem Dithiourethandisulfide, wie z.B. Tetramethylthiuramdisulfid oder Isopropylxanthogensäuredisulfid wirken bei der radikalischen Photopolymerisation als sogenannte Photoiniferter. Es handelt sich dabei um Verbindungen, die sowohl als Photoinitiator (Photoini-) wirken, als auch an Übertragungsreaktionen (engl.: transfer, -fer-) und Abbruchsreaktionen (engl.: termination, -ter) teilnehmen (vgl. T. Otsu, M. Yoshida, Makromol. Chem., Rapid. Commun. 3 (1982) 127-132: Role of Initiator-Transfer Agent-Terminator (Iniferter) in Radical Polymerizations: Polymer Design by Organic Disulfides as Iniferters). Die Zugabe von kettenübertragungsaktiven Stoffen, d.h. von Kettenreglern oder Photoinifertern, bewirkt eine Verringerung der Netzwerkdichte des Polyreaktionsnetzwerkes bei nahezu unveränderter Reaktivität der Polyreaktionsharzmischung. Kettenregler und Photoiniferter werden vorzugsweise in einer Menge von jeweils 0,005 bis 2 Gew.-% und besonders bevorzugt 0,01 bis 1,0 Gew.-% bezogen auf die Komponente **(A)** eingesetzt.

Als Entbinderungsbeschleuniger lassen sich erfindungsgemäß vorteilhaft auch Comonomere einsetzen, die zu einer Verringerung der thermische Stabilität von Polymernetzwerken führen. Hierzu eignen sich Comonomere, die thermisch labile Gruppen enthalten, wie z.B. Peroxid-, Azo- oder Urethangruppen, welche während des stereolithographischen Prozesses in das Polymernetzwerk eingebaut werden und dann im thermischen Entbinderungsprozess den Abbau des Polymernetzwerkes beschleunigen. Ein bevorzugtes Beispiel für ein polymerisationsfähiges Peroxid ist 4,4'-Divinylbenzoylperoxid, das durch Umsetzung von 4-Vinylbenzoylchlorid mit Natriumperoxid zugänglich ist. Ein bevorzugtes Beispiel für eine polymerisationsfähige Azoverbindung ist der Ester aus 2-Hydroxyethylmethacrylat und der 4,4'-Azobis-(4-cyano-valeriansäure). Bevorzugte thermisch labile Urethane sind aus Diisocyanaten zugänglich, beispielsweise durch Umsetzung von 2,2,4-Trimethylhexamethylendiisocyanat (TMDI) oder Toluylendiisocyanat (TDI) mit Hydroxypropylacrylat (HPA) oder 2-Hydroxyethylacrylat (HEA). Ein weiteres Beispiel für einen thermisch labilen Monomerbaustein stellt α,α,α',α'-Tetramethyl-1,4-benzen-dimethylacrylat dar, dessen Einbau in ein Michael-Addition-Netzwerke beispielsweise von Diacrylaten und Diacetoacetaten in Gegenwart katalytischer Säuremengen zu einem beschleunigten Abbau des Polymernetzwerks führt.

Außerdem sind als Entbinderungsbeschleuniger Comonomere geeignet, deren Polyreaktionsprodukte thermisch leicht abbaubar sind. Für radikalische Polymerisationsharze sind Comonomere bevorzugt, die wie α-Methylstyrol eine niedrige ceiling-Temperatur T_{c} aufweisen. Die ceiling-Temperatur ist die Grenztemperatur, bei der die Polymerisation mit der Depolymerisation im Gleichgewicht steht, und lässt sich aus dem Quotienten der Polymerisationsenthalpie und der Polymerisationsentropie berechnen (vgl. H.-G. Elias, Makromoleküle, Bd 1, 6. Aufl., Wiley-VCH, Weinheim etc. 1999, 193 ff.). Beispielsweise beträgt T_{c} für α-Methylstyrol 61 °C. Die ceiling-Temperatur T_{C} von Polytetrahydrofuran (PTHF) liegt bei 80 °C. Dementsprechend kann z.B. durch Verwendung von Telechelen mit radikalisch polymerisierbaren Gruppen, insbesondere PTHF-Di(meth)acrylat-Telechelen, als Comonomer die Abbaubarkeit von Poly(meth)acrylatnetzwerken beschleunigt werden. Erfindungsgemäß sind Comonomere mit einer ceiling-Temperatur von -10 bis 150 °C, vorzugsweise 10 bis 150 °C und besonders bevorzugt 20 bis 130 °C besonders geeignet. Die Comonomere werden vorzugsweise in einer Menge von 0,1 bis 30 Gew.-% und besonders bevorzugt 0,5 bis 20 Gew.% bezogen auf die Komponente **(A)** eingesetzt.

Als Komponente **F** enthalten die erfindungsgemäßen Schlicker vorzugsweise eine farbgebende Komponente. Die farbgebende Komponente wird vorzugsweise in einer Menge von 0,00001 bis 2,0 Gew.-%, besonders bevorzugt 0,001 bis 1,0 Gew.-% und ganz besonders bevorzugt 0,01 bis 0,5 Gew.-% eingesetzt, bezogen auf die Masse der Komponente **C.**

Als farbgebende Komponente sind die üblichen Farbstoffe oder Pigmente erfindungsgemäß nicht geeignet, da sie nicht genügend stabil sind, um den Entbinderungs- oder Sinterungsprozess zu überstehen. Erfindungsgemäß werden als Komponente F reaktive Übergangsmetallverbindungen eingesetzt, die einerseits im Bindemittel **A** löslich sind und den Verlauf der Photohärtung nicht nachteilig beeinflussen und die andererseits während der Entbinderung des stereolithographisch hergestellten Keramikgrünlings oder der Sinterung des daraus erhaltenen Keramikweisslings farbgebende Übergangsmetallionen bilden. Als farbgebende Komponente bevorzugte Übergangsmetallverbindungen sind vor allem Acetylacetonate oder Carbonsäuresalze der Elemente Eisen, Cer, Praseodym, Terbium, Lanthan, Wolfram, Osmium, Terbium und Mangan. Bevorzugt sind die Salze der Carbonsäuren Essig-, Propion-, Butter-, 2-Ethylhexylcarbon-, Stearin- und Palmitinsäure. Besonders bevorzugt sind vor allem die entsprechenden Fe-, Pr-, Mn- und Tb-Verbindungen, wie z.B. Eisen(III)-acetat oder -acetylacetonat, Mangan(III)-acetat oder -acetylacetonat, Praseodym(III)-acetat oder -acetylacetonat oder Terbium(III)-acetat oder -acetylacetonat sowie die entsprechenden Carbonsäuresalze.

Vorzugsweise werden die farbgebenden Komponenten so gewählt, dass nach dem Entbindern und Sintern zahnfarbene Keramikformteile erhalten werden.

Die als Komponente **F** verwendeten Übergangsmetallverbindungen werden als farbgebende Komponente (meist als Ionen) in das Gefüge eingebaut und beeinflussen nach dem Sinterprozess die Farbe der Sinterkörper. Im Gegensatz dazu dienen die vorgängig genannten organischen Farbstoffe zur Einstellung der Durchhärtungstiefe der Schlicker. Diese werden während der Entbinderung und Sinterung vollständig abgebaut. Erfindungsgemäß sind demgemäß solche Schlicker bevorzugt, die neben den Komponenten **A, B** und **C** einen Inhibitor **D,** einen Entbinderungsbeschleuniger **E** und/oder eine farbgebende Komponente **F** enthalten. Besonders bevorzugt sind Schlicker, welche die Komponenten **A, B, C** und **D; A, B, C** und **E; A, B, C** und **F** enthalten, ganz besonders bevorzugt sind Schlicker, welche die Komponenten **A, B, C, D** und **E; A, B, C, D** und **F; A, B, C, E** und **F** enthalten, und insbesondere Schlicker, welche die Komponenten **A, B, C, D, E** und **F** enthalten. Dabei ist es jeweils bevorzugt, die oben definierten Komponenten und bevorzugten Komponenten einzusetzen, vorzugsweise in den angegebenen Mengen.

Neben den Komponenten **A** bis **F** können die erfindungsgemäßen Schlicker weitere Komponenten als Additive enthalten.

Beispielsweise können die erfindungsgemäßen Schlicker einen oder mehrere Dispergatoren enthalten, die die Bildung von Agglomeraten und das Absetzen der Keramikpartikel verhindern. Geeignete Dispergatoren für Keramikschlicker sind vor allem Polymere wie Polycarbonsäuren, Polycarbonsäuresalze oder nichtionische Polymere, wie z.B. Polyethylenglycol oder Carboxymethylcellulose. Dabei eignen sich als Dispergatoren vor allem Polymere, die im Polyreaktionsharz löslich sind. Beispielsweise Poly(meth)acrylate, die in lichthärtenden (Meth)acrylatharzen löslich sind, wobei mit zunehmender Molmasse und Gehalt an diesen Polymeren im Schlicker der erreichbare Feststoffgehalt in der Regel abnimmt. Weiterhin können die Partikel der Komponente **C** auch schon vor der Schlickerherstellung mit den Dispergatoren vorbehandelt werden. Dispergatoren werden vorzugsweise in einer Menge von 0 bis 5 Gew.-% und besonders bevorzugt 0,5 bis 3,0 Gew.-% eingesetzt, bezogen auf die Gesamtmasse des Schlickers.

Als weitere Komponenten können die erfindungsgemäßen Keramikschlicker einen oder mehreren Weichmacher enthalten, die verhindern, dass der Keramikgrünling nach der photochemischen Aushärtung und einem evt. Trocknen brüchig wird. Darüber hinaus sorgen Weichmacher für eine ausreichende Flexibilität. Typische Weichmacher sind Phthalate, wie z.B. Dibutyl- oder Dihexylphthalat, nichtsaure Phosphate, wie z.B. Tributyl- oder Trikresylphosphat, n-Octanol, Glycerin oder Polyethylen- bzw. Polypropylenglykole bzw. entsprechende Ester- oder Etherderivate. Weichmacher werden vorzugsweise in einer Menge von 0 bis 20 Gew.-% und besonders bevorzugt 0 bis 10 Gew.-% eingesetzt, bezogen auf die Gesamtmasse an radikalisch polymerisierbaren Komponenten.

Weiterhin können die erfindungsgemäßen Schlicker vorteilhaft auch ein Lösungsmittel enthalten. Als Lösungsmittel eignen sich beispielsweise die oben als Weichmacher genannten Verbindungen.

Vorzugsweise werden als Lösungsmittel solche Komponenten eingesetzt, die eine Siedetemperatur von mindestens ca. 120 °C und eine relativ hohe Verdampfungsenthalpie besitzen, so dass es bei der stereolithographischen Verarbeitung des Schlickers nicht zur vorzeitigen Verdunstung kommt. Besonders bevorzugt sind Lösungsmittel mit einer Siedetemperatur von 150 bis 250 °C, ganz besonders bevorzugt von 180 bis 230 °C. Besonders geeignet sind 1-Octanol, 1- oder 2-Nonanol, Diethylenglycoldiethylether, 2,5-Dimethoxytetrahydrofuran, Oxalsäuredibutylester, Cyclohexanol, Polypropylenglycoldiacetat, Cyclohexanon, Acetessigsäureethylester und Mischungen davon. Das oder die Lösungsmittel werden vorzugsweise in einer Menge von 0 bis 70 Gew.-%, besonders bevorzugt von 10 bis 50 Gew.-% eingesetzt, bezogen auf die Gesamtmasse an radikalisch polymerisierbaren Komponenten.

Als Weichmacher und/oder Lösungsmittel sind Mischungen solcher Verbindungen besonders geeignet, die in einem Temperaturbereich zwischen 150 und 250 °C schrittweise verdampfbar sind. Schrittweise verdampfbar bedeutet hier, dass man Mischungen von einem oder mehreren Weichmachern und/oder einem oder mehreren Lösungsmitteln einsetzt, die unterschiedliche Siedetemperaturen aufweisen, so dass sich diese Komponenten nacheinander verdampfen lassen, vorzugsweise in einem Temperaturbereich von 150°C bis 250°C, und damit wesentlich schonender für den Formkörper entfernt werden können.

Das Verdampfen der Lösungsmittel kann das bei höherer Temperatur stattfindende Entbindern der Polymeranteile begünstigen. Es wurde gefunden, dass das Verdampfen der obigen Lösungsmittel zur Bildung von Mikroporen im Grünling führt, die sich beim Sintern wieder schließen, die aber das Entweichen der Gase im Entbinderungsschritt ermöglichen und so das Entstehen von Spannungen und Rissen verhindern. Außerdem wird die Gefahr einer Trennung der stereolithographisch erzeugten Schichten reduziert und eine vollständige Entfernung der organischen Komponenten erleichtert.

Alternativ kann eine Porosität des Grünlings auch dadurch erreicht werden, dass vor Wärmebehandlung herauswaschbare Anteile extraktiv entfernt werden. Geeignete extrahierbare Komponenten sind wasserlösliche Polymere, wie z.B. Polyvinylalkohol, Polyvinylpyrrolidon und Polyethylenglycole. Weiterhin können benzinlösliche Stoffe wie Parafine oder Wachse und langkettige Fettsäureester eingesetzt werden. Die bevorzugte Menge an extrahierbaren Komponenten in der Harzmatrix liegt zwischen 0 und 40 Gew.-%, besonders bevorzugt zwischen 0,1 und 30 Gew.-%, bezogen auf die Komponente **(A).**

Außerdem können die erfindungsgemäß Schlicker Entschäumungsmittel und/oder Hautverhinderungsmittel enthalten, die die Schaumbildung bei der Herstellung der Schlicker bzw. die Bildung einer Haut während der Verarbeitung der Schlicker verhindern. Entschäumungs- und/oder Hautverhinderungsmittel werden vorzugsweise in eine Menge von jeweils 0 bis 5 Gew.-%, besonders bevorzugt 0,1 bis 2 Gew.-% in der organischen Matrix eingesetzt, bezogen auf die Masse der Komponente **(A).**

Erfindungsgemäß besonders bevorzugt sind Schlicker, welche die oben genannten Komponenten in folgenden Mengen enthalten:

| | |
|---|---|
| Komponente **A:** | 1 bis 30, bevorzugt 5 bis 20 Gew.-% |
| Komponente **A2:** | 0 bis 20, bevorzugt 0 bis 10 Gew.-% |
| Komponente **B:** | 0,001 bis 2, bevorzugt 0,01 bis 1,0 Gew.-% |
| Komponente **C:** | 30 bis 90, besonders bevorzugt 35 bis 85 und ganz besonders bevorzugt 40 bis 85 Gew.-% |
| Lösungsmittel und/oder | |
| Weichmacher | 0 bis 30, bevorzugt 0 bis 15 Gew.-% |

Die rheologischen Eigenschaften der erfindungsgemäßen Schlicker werden vorzugsweise so eingestellt, dass ihre Viskosität im Bereich von 0,02-20.000 Pa·s, besonders bevorzugt 0,05-5.000 Pa·s liegt. Dabei ist es von Vorteil, wenn möglichst keine Fliessgrenzen auftreten. Die Viskosität und die Fliessgrenze werden bei 23 °C mit einem Platte-Platte-Viskosimeter bestimmt.

Die erfindungsgemäßen Schlicker eignen sich besonders zur Herstellung von Keramik- oder Glaskeramikformteilen, insbesondere zur Herstellung von Dentalrestaurationen, wie z.B. Inlays, Onlays, Veneers, Kronen, Brücken oder Gerüsten.

Gegenstand der Erfindung ist auch ein Verfahren zur Herstellung von Keramik- oder Glaskeramikformteilen bei dem man
(a) einen Grünling herstellt, indem man einen erfindungsgemäßen Schlicker durch lokales Einbringen von Strahlungsenergie unter Ausbildung der geometrischen Form des Grünlings aushärtet,
(b) man dann den Grünling einer Wärmebehandlung zur Entfernung des Bindemittels (Entbinderung) unterwirft, um einen Weißling zu erhalten, und
(c) man den Weißling anschließend sintert.

Die Herstellung des Grünlings in Schritt (a) erfolgt durch Rapid Prototyping, vorzugsweise durch Stereolithographie. Es wird durch schichtweise Strahlungshärtung eines fließfähigen keramischen Schlickers ein keramischer Grünling hergestellt, der in Schritt (b) entbindert wird. Hierbei wird das verwendete Bindemittel durch Erhitzen des Grünlings auf eine Temperatur von vorzugsweise 90 °C bis 600 °C entfernt, und es wird der sogenannte Weißling erhalten. Der Weißling wird in Schritt (c) zu einem dichten keramischen Formkörper gesintert. Die Sinterung des Weißlings erfolgt im Sinterofen, vorzugsweise bei einer Temperatur für Glaskeramik von 650 bis 1100 °C, bevorzugt 700 bis 900 °C, für Zirkondioxid von 1100 bis 1600 °C, bevorzugt 1400 bis 1500 °C und für Aluminiumoxid von 1400 bis 1800°C, bevorzugt 1600 bis 1700°C. Die nach dem erfindungsgemäßen Verfahren hergestellten Keramikformkörper zeichnen sich durch eine hohe Festigkeit und große Detailgenauigkeit aus. Die Biegefestigkeit nach ISO 6872 liegt bei Formkörpern aus Glaskeramik vorzugsweise über 100 MPa, insbesondere im Bereich von 150 bis 500 MPa. Formkörper aus Al₂O₃ haben eine Biegefestigkeit von vorzugsweise über 300 MPa, insbesondere von 500 bis 700 MPa, Formkörper aus ZrO₂ von mehr als 500 MPa insbesondere von 800 bis 1100 MPa.

Die Erfindung wird im Folgenden anhand von Zeichnungen und Ausführungsbeispielen näher erläutert.

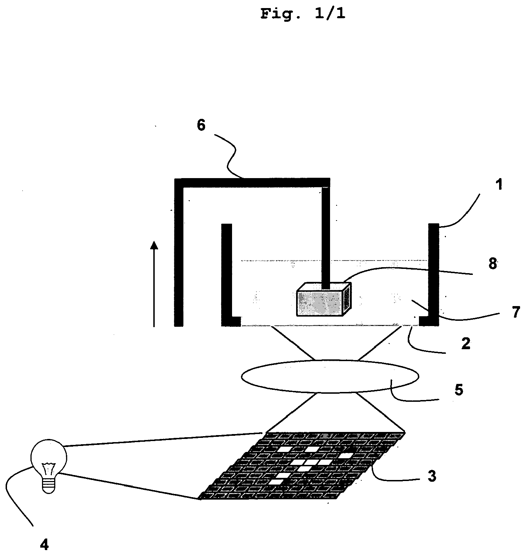

**Fig. 1** zeigt eine schematische Darstellung einer Vorrichtung zur Durchführung des erfindungsgemäßen Verfahrens. Wie Fig. 1 zu entnehmen ist, umfasst die Vorrichtung einen Behälter **1** für den erfindungsgemäßen Schlicker **7.** Der Behälter **1** wird auch als Polymerisationswanne oder Wanne bezeichnet. In der dargestellten Ausführungsform weist die Wanne **1** ein transparentes Fenster **2** auf, durch das der Schlicker von unten selektiv belichtet und ausgehärtet wird. Unterhalb der Wanne **1** ist ein computergesteuerter, beweglicher Spiegel **3,** ein sogenanntes Mikrospiegelfeld (micro-mirror array), angeordnet, das mit einer Strahlungsquelle **4** bestrahlt wird. Das Bild des Spiegels **3** wird durch eine optische Vorrichtung **5** auf das transparente Fenster **2** projiziert. Über der Wanne **1** ist ein in Z-Richtung verfahrbarer Substratträger **6** angeordnet, der den schichtweise aufgebauten Körper **8** trägt. Der Substratträger **6** kann eine hier nicht dargestellte Trägerplatte aufweisen. Der Substratträger **6** wird soweit in den Schlicker eingetaucht, dass der Abstand zwischen dem Träger **6** bzw. dem daran befestigen Körper **8** und der inneren Oberfläche der Wanne **1** der Schichtdicke der zu erzeugenden Schicht entspricht. Anschließend wird die Schlickerschicht zwischen Träger **6** und innerer Wannenoberfläche durch das transparente Fenster **2** hindurch mit Hilfe des Spiegels **3** selektiv belichtet und gehärtet. Es entstehen gehärtete Bereiche, die an dem Träger **6** anhaften. Anschließend wird der Träger **6** in Z-Richtung nach oben bewegt, so dass zwischen der anhaftenden Schicht und der inneren Wannenoberfläche wieder eine Schlickerschicht mit der gewünschten Dicke entsteht. Durch erneute Belichtung wird auch diese Schicht selektiv gehärtet und durch Wiederholung des Vorgangs der gewünschte Formkörper, vorzugsweise eine dentale Restauration, schichtweise aufgebaut.

### Ausführungsbeispiele

### Beispiel 1

### Lichthärtende Keramikschlicker und stereolithographisch erzeugte Formkörper

Zur Herstellung der Keramikschlicker wurden die in der nachfolgenden Tabelle aufgeführten Monomeren (PS-m-HEA = Umsetzungsprodukt von 1 mol Phthalsäureanhydrid mit 1 mol 2-Hydroxyethylacrylat; NK-Ester CBX-1N = Pentaerithritoltriacrylatmonophthalat 70%), die Lösungsmittel PEG-400 bzw. PPG-400 sowie ein Dispergator vorgelegt und homogen vermischt. Danach wurde der Photoinitiator K-69 (Bis(4-methoxybenzoyl)di-ethylgermanium, Ivoclar Vivadent AG) oder Irgacure 819 (Bis(2,4,6-trimethylbenzoyl)phenylphosphinoxid, Ciba SC) zugeben und in der Mischung durch einfaches Rühren gelöst. Schließlich wurde das Zirkonoxidpulver TZ-3YS-E (Firma Tosoh) portionsweise zugegeben und dispergiert. Es bildeten sich fließfähige, homogene, stabile Schlicker mit einem Füllgrad von ca. 37 Volumen-%.

Mit den Schlickern A und B wurden auf einer SL-Anlage Biaxial-Prüfkörper generativ gefertigt. Die Prüfkörper wurden gereinigt und danach gesintert.

Die gesinterten Biaxialplättchen wurden gemäß ISO 6872:2008 präpariert und gemessen. Für den Schlicker A bzw. B ergaben sich Keramikformkörper mit einer Biaxial-Festigkeit von 1043 bzw. 1055 MPa

| **Komponente** | **A [Gew.-%)** | **B [Gew.-%]** |
|---|---|---|
| Dispergator | 1,00 | 1,00 |
| PS-m-HEA | 12,00 | 11,00 |
| NK-Ester CBX-1N | - | 1,00 |
| PEG 400 | 9,95 | - |
| PPG 400 | - | 9,75 |
| K-69 | 0,05 | - |
| Irgacure 819 | - | 0,25 |
| TZ-3YS-E Tosoh | 77,00 | 77,00 |
| Summe | 100,00 | 100,00 |

## Patentansprüche

1. Schlicker auf der Basis von radikalisch polymerisierbarem Bindemittel, Polymerisationsinitiator und Füllstoff, **dadurch gekennzeichnet, dass** er die folgenden Komponenten enthält:
**A)** 1 bis 30 Gew.-% mindestens eines aciden Monomers der allgemeinen Formel I 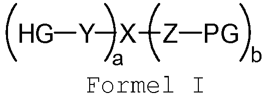 in der
X entfällt oder ein (a+b)-wertiger, substituierter oder unsubstituierter, geradkettiger aliphatischer Rest mit mindestens 2, ein verzweigter aliphatischer Rest mit mindestens 3, ein cycloaliphatischer, bi- oder tricylischer oder aromatischer Rest mit 6 bis 20 Kohlenstoffatomen oder eine Kombination dieser Reste ist, wobei diese Gruppen 1 oder mehrere Ethergruppen enthalten können,
PG eine radikalisch polymerisierbare Gruppe, ist,
HG -COOH ist,
Y entfällt oder eine zweiwertige, geradkettige aliphatische Gruppe mit 2 bis 40 Kohlenstoffatomen, eine verzweigte aliphatische Gruppe mit 3 bis 40 Kohlenstoffatomen oder eine cycloaliphatische oder aromatische Gruppe mit 5 bis 40 Kohlenstoffatomen ist, wobei Y eine oder mehrere Ether-, Thioether-, Amid- und/oder Estergruppen enthalten kann,
Z entfällt oder eine zweiwertige, geradkettige aliphatische Gruppe mit 2 bis 40 Kohlenstoffatomen oder eine verzweigte aliphatische Gruppe mit 3 bis 40 Kohlenstoffatomen ist, wobei Z eine oder mehrere Ether-, Thioether-, Amid- und/oder Estergruppen enthalten kann,
a eine ganze Zahl von 1 bis 5 ist; und
b 1 oder 2 ist;
**A2)** 0 bis 50 Gew.-% mindestens eines nicht aciden radikalisch polymerisierbaren Monomers;
**B)** 0,001 bis 2,0 Gew.-% Photoinitiator; und
**C)** 30 bis 90 Gew.-% Keramik- und/oder Glaskeramikpartikel;
jeweils bezogen auf die Gesamtmasse des Schlickers;
wobei der Schlicker als Komponente **(C)** Glaskeramikpartikel oder Keramikpartikel auf der Basis von reinem ZrO₂, reinem Al₂O₃, reinem ZrO₂-Al₂O₃, ZrO₂, das mit HfO₂, CaO, Y₂O₃, CeO₂ und/oder MgO stabilisiert ist, ZrO₂-Al₂O₃, das mit HfO₂, CaO, Y₂O₃, CeO₂ und/oder MgO stabilisiert ist, oder Nicht-Oxid-Keramiken enthält.

2. Schlicker nach Anspruch 1, bei dem die Variablen der Formel(I) folgende Bedeutung haben:
X entfällt oder ein (a+b)-wertiger, substituierter oder unsubstituierter, geradkettiger aliphatischer Rest mit 3 bis 15 Kohlenstoffatomen, ein verzweigter aliphatischer Rest mit 3 bis 15 Kohlenstoffatomen, ein cycloaliphatischer, bi- oder tricylischer oder aromatischer Rest mit 6 bis 15 Kohlenstoffatomen oder eine Kombination dieser Reste, vorzugsweise ein aromatischer oder cycloaliphatischer C₆-Rest und insbesondere ein geradkettiger oder verzweigter aliphatischer C₃-C₁₅-Rest, besonders bevorzugt C₃-C₈-Rest, wobei diese Gruppen 1 oder mehrere Ethergruppen enthalten können;
PG eine Vinyl- (CH₂=CH-), Allyl- (CH₂=CH-CH₂-), Allylether- (CH₂=CH-CH₂-O-), Styryl (CH₂=CH-Phenyl-), (Meth)acrylat- (CH₂=C(-H/-CH₃) -CO-O-) oder (Meth)-acrylamid-Gruppe (CH₂=C(-H/-CH₃)-CO-NH- oder CH₂=C(-H/-CH₃) -CO-NR- mit R= C₁-C₄-Alkyl), besonders bevorzugt eine (Meth)acrylat- oder (Meth)acrylamid-Gruppe und ganz besonders bevorzugt eine Acrylat-(CH₂=CH-CO-O-) oder Acrylamid-Gruppe (CH₂=CH-CO-NH-);
HG -COOH;
Y entfällt oder ein geradkettiger aliphatischer Rest mit 2 bis 10 Kohlenstoffatomen, ein verzweigter aliphatischer Rest mit 3 bis 12 Kohlenstoffatomen, ein cycloaliphatischer, bi- oder tricylischer oder aromatischer Rest mit 6 bis 15 Kohlenstoffatomen oder eine Kombination dieser Reste;
Z entfällt oder ein geradkettiger aliphatischer Rest mit 2 bis 10 Kohlenstoffatomen, ein verzweigter aliphatischer Rest mit 3 bis 12 Kohlenstoffatomen, ein cycloaliphatischer, bi- oder tricylischer oder aromatischer Rest mit 6 bis 15 Kohlenstoffatomen, eine Kombination dieser Reste oder ein Oligoethylenglycol-(-(CH₂-CH₂-O)ₙ-, n = 1-5) oder ein Oligopropylenglycolrest (-(CH(CH₃)-CH₂-O)ₙ-, n = 1-5);
a 1, 2 oder 3; und
b 1.

3. Schlicker nach Anspruch 2, bei dem die Variablen der Formel(I) folgende Bedeutung haben:
X entfällt oder ein (a+b)-wertiger, unsubstituierter, verzweigter oder geradkettiger aliphatischer Rest mit 3 bis 15, vorzugsweise 3 bis 8 Kohlenstoffatomen, der 1 oder 2 Ethergruppen enthalten kann;
PG eine Acrylat- oder Acrylamid-Gruppe,
HG -COOH,
Y eine zweiwertige, geradkettige aliphatische Gruppe mit 2 bis 8 Kohlenstoffatomen oder eine verzweigte aliphatische Gruppe mit 3 bis 8 Kohlenstoffatomen, wobei diese Gruppe 1 Estergruppe enthalten kann, und
Z eine zweiwertige, geradkettige aliphatische Gruppe mit 2 bis 10 Kohlenstoffatomen oder eine verzweigte aliphatische Gruppe mit 3 bis 10 Kohlenstoffatomen, wobei diese Gruppen 1 oder mehrere, vorzugsweise 0 bis 3 Ethergruppen enthalten kann,
a 1, 2 oder 3, und
b 1.

4. Schlicker nach einem der Ansprüche 1 bis 3, der zusätzlich
**D)** 0,001 bis 1,0 Gew.-% Inhibitor
enthält, bezogen auf die Gesamtmasse des Schlickers.

5. Schlicker nach einem der Ansprüche 1 bis 4, der zusätzlich
0 bis 20 Gew.-% Entbinderungsbeschleuniger **(E),**
0 bis 20 Gew.-% Weichmacher und
0 bis 70 Gew.-% Lösungsmittel enthält,
jeweils bezogen auf die Gesamtmasse an radikalisch polymerisierbaren Komponenten.

6. Schlicker nach einem der Ansprüche 1 bis 5, der zusätzlich
**F)** 0,00001 bis 2,0 Gew.-% farbgebende Komponenten enthält, bezogen auf die Masse der Komponente (C).

7. Schlicker nach einem der Ansprüche 1 bis 6, bei dem die Partikel der Komponente **(C)** mit einer linearen oder verzweigten Carbonsäure, insbesondere Ameisensäure, Essigsäure, Propionsäure, Octansäure, Isobuttersäure, Isovaleriansäure oder Pivalinsäure; einem sauren Phosphorsäureester, insbesondere Dimethyl-, Diethyl-, Dipropyl-, Dibutyl-, Dipentyl-, Dihexyl-, Dioctyl- oder Di(2-ethylhexyl)phosphat; einer Phosphonsäure, insbesondere Methyl-, Ethyl-, Propyl-, Butyl- Hexyl-, Octyl- oder Phenylphosphonsäure; oder einem Silan, insbesondere Propyltrimethoxysilan, Phenyltrimethoxysilan, Hexyltrimethoxysilan, Octyltrimethoxysilan, Trimethylchlorsilan, Trimethylbromsilan, Trimethylmethoxysilan oder Hexamethyldisilazan, oberflächenmodifiziert sind, wobei das Oberflächenmodifizierungmittel keine radikalisch polymerisierbaren Gruppen aufweist.

8. Schlicker nach einem der Ansprüche 1 bis 6, bei dem die Partikel der Komponente **(C)** eine Partikelgröße im Bereich von 20 nm bis 50 µm aufweisen.

9. Schlicker nach einem der Ansprüche 5 bis 8, der als Entbinderungsbeschleuniger **(E)**
- einen Kettenregler, insbesondere ein Mercaptan, ein Disulfid oder einen Photoiniferter, vorzugsweise Laurylmercaptan, ein Dithiourethandisulfid, Tetramethylthiuramdisulfid oder Isopropylxanthogensäuredisulfid; und/oder
- ein Comonomer, das eine oder mehrere thermisch labile Gruppen, insbesondere eine oder mehrere Peroxid-, Azo- oder Urethangruppen aufweist; und/oder
- ein Comonomer mit einer ceiling-Temperatur von -10 bis 150 °C, insbesondere α-Methylstyrol, Polytetrahydrofuran (PTHF), oder ein Telechel mit radikalisch polymerisierbaren Gruppen, insbesondere ein PTHF-Di(meth)acrylat-Telechel
enthält.

10. Schlicker nach einem der Ansprüche 6 bis 9, der als farbgebende Komponente **(F)** eine Übergangsmetallverbindung enthält, insbesondere ein Acetylacetonat und/oder ein Carbonsäuresalz der Elemente Eisen, Cer, Praseodym, Terbium, Lanthan, Wolfram, Osmium, Terbium und Mangan, insbesondere Eisen(III)-acetat oder Eisen(III)-acetylacetonat, Mangan(III)-acetat oder Mangan(III)-acetylacetonat, Praseodym(III)-acetat oder Praseodym(III)-acetylacetonat oder Terbium(III)-acetat oder Terbium(III)-acetylacetonat.

11. Schlicker nach einem der Ansprüche 1 bis 10, der zusätzlich mindestens eine weitere Komponente aus der Gruppe Dispergator, Entschäumungsmittel, Hautverhinderungsmittel enthält.

12. Schlicker nach einem der Ansprüche 1 bis 11, der maximal 40 Gew.-% an multifunktionellen Monomeren bezogen auf die Gesamtmasse an radikalisch polymerisierbaren Komponenten enthält.

13. Verwendung eines Schlickers gemäß einem der Ansprüche 1 bis 12 zur Herstellung von Keramik- oder Glaskeramikformteilen.

14. Verwendung nach Anspruch 13, wobei das Keramikformteil eine Dentalrestauration ist, wie z.B. ein Inlay, Onlay, Veneer, eine Krone, Brücke oder ein Gerüst.

15. Verfahren zur Herstellung eines Keramik- oder Glaskeramikformteils bei dem man
(a) einen Grünling herstellt, indem man einen Schlicker gemäß einem der Ansprüche 1 bis 10 durch lokales Einbringen von Strahlungsenergie unter Ausbildung der geometrischen Form des Grünlings aushärtet,
(b) man dann den Grünling einer Wärmebehanldung zur Entfernung des Bindemittels (Entbinderung) unterwirft, um einen Weißling zu erhalten, und
(c) man den Weißling sintert.

## Claims

1. Slip based on a radically polymerisable binder, polymerisation initiator and filler, **characterised in that** it comprises the following components:
**A)** 1 to 30 wt % of at least one acidic monomer of the general Formula I 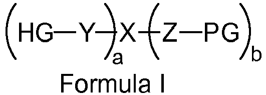 in which
X is missing or is an (a+b)-valent, substituted or unsubstituted, straight chain aliphatic group with at least 2 carbon atoms, a branched aliphatic group with at least 3 carbon atoms, a cycloaliphatic, bi- or tricylic or aromatic group with 6 to 20 carbon atoms or a combination of these groups, wherein these groups can contain one or more ether groups,
PG is a radically polymerisable group;
HG is -COOH,
Y is missing or is a divalent, straight chain aliphatic group with 2 to 40 carbon atoms, a branched aliphatic group with 3 to 40 carbon atoms or a cycloaliphatic or aromatic group with 5 to 40 carbon atoms, wherein Y can contain one or more ether, thioether, amide and/or ester groups,
Z is missing or is a divalent, straight chain aliphatic group with 2 to 40 carbon atoms or a branched aliphatic group with 3 to 40 carbon atoms, wherein Z can contain one or more ether, thioether, amide and/or ester groups,
a is an integer from 1 to 5; and
b is 1 or 2;
**A2)** 0 to 50 wt % of at least one non-acidic radically polymerisable monomer;
**B)** 0.001 to 2.0 wt % photo initiator; and
**C)** 30 to 90 wt % ceramic and/or glass ceramic particles;
each relative to the total weight of the slip;
wherein the slip comprises as component (C) glass ceramic particles or ceramic particles based on pure ZrO₂, pure Al₂O₃, pure ZrO₂-Al₂O₃, ZrO₂ that is stabilised with HfO₂, CaO, Y₂O₃, CeO₂ and/or MgO, ZrO₂-Al₂O₃ that is stabilised with HfO₂, CaO, Y₂O₃, CeO₂ and/or MgO, or non-oxide ceramics.

2. Slip according to claim 1, wherein the variables of Formula (I) have the following meaning:
X is missing or is an (a+b)-valent, substituted or unsubstituted, straight chain aliphatic group with 3 to 15 carbon atoms, a branched aliphatic group with 3 to 15 carbon atoms, a cycloaliphatic, bi- or tricylic or aromatic group with 6 to 15 carbon atoms or a combination of these groups, preferably an aromatic or cycloaliphatic C₆ group and in particular is a straight chain or branched aliphatic C₃-C₁₅ group, particularly preferably a C₃-C₈ group, wherein these groups can contain one or more ether groups;
PG is a vinyl (CH₂=CH-), allyl (CH₂=CH-CH₂-), allyl ether (CH₂=CH-CH₂-O-), styryl (CH₂=CH-phenyl-), (meth)acrylate (CH₂=C(-H/-CH₃)-CO-O-) or (meth)acrylamide group (CH₂=C(-H/-CH₃)-CO-NH- or CH₂=C(-H/-CH₃)-CO-NR- with R = C₁-C₄ alkyl), particularly preferably a (meth)acrylate or (meth)acrylamide group and quite particularly preferably an acrylate (CH₂=CH-CO-O-) or acrylamide group (CH₂=CH-CO-NH-);
HG is -COOH;
Y is missing or is a straight chain aliphatic group with 2 to 10 carbon atoms, a branched aliphatic group with 3 to 12 carbon atoms, a cycloaliphatic, bi- or tricylic or aromatic group with 6 to 15 carbon atoms or a combination of these groups;
Z is missing or is a straight chain aliphatic group with 2 to 10 carbon atoms, a branched aliphatic group with 3 to 12 carbon atoms, a cycloaliphatic, bi- or tricylic or aromatic group with 6 to 15 carbon atoms, a combination of these groups or an oligomeric ethylene glycol (-(CH₂-CH₂-O)ₙ-, n = 1-5) or an oligomeric propylene glycol group (-(CH(CH₃)-CH₂-O)ₙ-, n = 1-5);
a is 1, 2 or 3; and
b is 1.

3. Slip according to claim 2, wherein the variables of Formula (I) have the following meaning:
X is missing or is an (a+b)-valent, unsubstituted, branched or straight chain aliphatic group with 3 to 15, preferably 3 to 8 carbon atoms, which can contain 1 or 2 ether groups;
PG is an acrylate or acrylamide group;
HG is -COOH;
Y is a divalent, straight chain aliphatic group with 2 to 8 carbon atoms or a branched aliphatic group with 3 to 8 carbon atoms, wherein this group can contain 1 ester group and
Z is a divalent, straight chain aliphatic group with 2 to 10 carbon atoms or a branched aliphatic group with 3 to 10 carbon atoms, wherein this group can contain 1 or more, preferably 0 to 3, ether groups,
a is 1, 2 or 3; and
b is 1.

4. Slip according to any one of claims 1 to 3, which further comprises
**D)** 0.001 to 1.0 wt % inhibitor, relative to the total weight of the slip.

5. Slip according to one of claims 1 to 4 which further comprises
0 to 20 wt % debonding accelerator **(E),**
0 to 20 wt % plasticiser and
0 to 70 wt % solvent,
each relative to the total weight of the radically polymerisable components.

6. Slip according to one of claims 1 to 5 further comprising
**F)** 0.00001 to 2.0 wt % colouring components, relative to the weight of component **(C).**

7. Slip according to one of claims 1 to 6, wherein the particles of component **(C)** are surface-modified with a linear or branched carboxylic acid, in particular formic acid, acetic acid, propionic acid, octanoic acid, *iso*butyric acid, *iso*valeric acid or pivalic acid; an acidic phosphoric acid ester, in particular dimethyl, diethyl, dipropyl, dibutyl, dipentyl, dihexyl, dioctyl or di(2-ethylhexyl) phosphate; a phosphonic acid, in particular methyl, ethyl, propyl, butyl, hexyl, octyl or phenyl phosphonic acid; or a silane, in particular propyltrimethoxysilane, phenyltrimethoxysilane, hexyltrimethoxysilane, octyltrimethoxysilane, chloro(trimethyl)silane, bromo(trimethyl)silane, methoxy(trimethyl)silane or hexamethyldisilazane, wherein the surface-modification agent does not possess radically polymerisable groups.

8. Slip according to one of claims 1 to 6, wherein the particles of component **(C)** have a particle size in the range from 20 nm to 50 µm.

9. Slip according to one of claims 5 to 8 comprising as the debinding accelerator **(E)**
- a chain transfer agent, in particular a mercaptan, a disulfide or a photoiniferter, preferably lauryl mercaptan, a dithiourethane disulfide, tetramethylthiuram disulfide or isopropyl xanthogenic acid disulfide; and/or
- a comonomer which has one or more thermally labile groups, in particular one or more peroxide, azo or urethane groups; and/or
- a comonomer with a ceiling temperature of -10 to 150 °C, in particular α-methylstyrene, polytetrahydrofuran (PTHF), or a telechelic with radically polymerisable groups, in particular a PTHF di(meth)acrylate telechelic.

10. Slip according to one of claims 6 to 9 comprising as the colouring component **(F)** a transition metal compound, in particular an acetylacetonate and/or a carboxylic acid salt of the elements iron, cerium, praseodymium, terbium, lanthanum, tungsten, osmium, terbium and manganese, in particular iron(III) acetate or iron(III) acetylacetonate, manganese(III) acetate or manganese(III) acetylacetonate, praseodymium(III) acetate or praseodymium(III) acetylacetonate or terbium(III) acetate or terbium(III) acetylacetonate.

11. Slip according to one of claims 1 to 10, additionally comprising at least one further component from the group of dispersants, defoaming agents, and antiskinning agents.

12. Slip according to one of claims 1 to 11, comprising at most 40 wt % of multifunctional monomers relative to the total weight of the radically polymerisable components.

13. Use of a slip according to one of claims 1 to 12 for the preparation of ceramic or glass ceramic shaped articles.

14. Use according to claim 13, wherein the ceramic shaped article is a dental restoration, such as e.g. an inlay, onlay, veneer, crown, bridge or framework.

15. Process for the preparation of a ceramic or glass ceramic shaped article, wherein
(a) a green body is prepared by curing a slip according to any one of claims 1 to 10 by locally introducing radiation energy to form the geometric shape of the green body,
(b) the green body is then subjected to a heat treatment to remove the binder (debonding), in order to obtain a white body, and
(c) the white body is sintered.

## Revendications

1. Barbotine à base de liant polymérisable par voie radicalaire, d'amorceur de polymérisation et de charge, **caractérisée en ce qu'**elle contient les composants suivants :
A) de 1 à 30 % en poids d'au moins un monomère acide de formule générale I 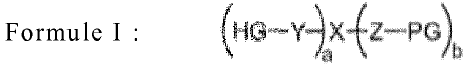 dans laquelle
- X ne représente rien ou représente un groupe de valence (a+b), avec ou sans substituant(s), de type aliphatique à chaîne linéaire comportant au moins 2 atomes de carbone, aliphatique à chaîne ramifiée comportant au moins 3 atomes de carbone, cycloaliphatique, bicyclique ou tricyclique ou aromatique comportant de 6 à 20 atomes de carbone, ou une combinaison de tels groupes, étant entendu que ces groupes peuvent comporter un ou plusieurs groupe(s) de type éther,
- PG représente un groupe polymérisable par voie radicalaire,
- HG représente un groupe carboxyle (-COOH),
- Y ne représente rien ou représente un groupe divalent, de type aliphatique à chaîne linéaire comportant de 2 à 40 atomes de carbone, aliphatique à chaîne ramifiée comportant de 3 à 40 atomes de carbone, ou cycloaliphatique ou aromatique comportant de 5 à 40 atomes de carbone, étant entendu que ce groupe représenté par Y peut comporter un ou plusieurs groupe(s) de type éther, thioéther, amide et/ou ester,
- Z ne représente rien ou représente un groupe divalent, de type aliphatique à chaîne linéaire comportant de 2 à 40 atomes de carbone ou aliphatique à chaîne ramifiée comportant de 3 à 40 atomes de carbone, étant entendu que ce groupe représenté par Z peut comporter un ou plusieurs groupe(s) de type éther, thioéther, amide et/ou ester,
- l'indice a est un nombre entier valant de 1 à 5,
- et l'indice b vaut 1 ou 2 ;
A2) de 0 à 50 % en poids d'au moins un monomère non-acide, polymérisable par voie radicalaire ;
B) de 0,001 à 2,0 % en poids de photoamorceur ;
C) et de 30 à 90 % en poids de particules de céramique et/ou de vitrocéramique ;
étant entendu que chaque pourcentage est rapporté à la masse totale de la barbotine,
et que la barbotine contient, en tant que composant (C), des particules de vitrocéramique ou des particules de céramique à base de zircone (ZrO₂) pure, d'alumine (Al₂O₃) pure, de zircone-alumine pure, de zircone stabilisée avec HfO₂, CaO, Y₂O₃, CeO₂ et/ou MgO, de zircone-alumine stabilisée avec HfO₂, CaO, Y₂O₃, CeO₂ et/ou MgO, ou de céramique non-oxyde.

2. Barbotine conforme à la revendication 1, dans laquelle les symboles de la formule I ont les significations suivantes :
- X ne représente rien ou représente un groupe de valence (a+b), avec ou sans substituant(s), de type aliphatique à chaîne linéaire comportant de 3 à 15 atomes de carbone, aliphatique à chaîne ramifiée comportant de 3 à 15 atomes de carbone, cycloaliphatique, bicyclique ou tricyclique ou aromatique comportant de 6 à 15 atomes de carbone, ou une combinaison de tels groupes, et de préférence, un groupe aromatique ou cycloaliphatique en C₆ et en particulier un groupe aliphatique à chaîne linéaire ou ramifiée en C₃-C₁₅, étant entendu que ces groupes peuvent comporter un ou plusieurs groupe(s) de type éther,
- PG représente un groupe vinyle (CH₂=CH-), allyle (CH₂=CH-CH₂-), allyl-oxy (CH₂=CH-CH₂-O-), styryle (CH₂=CH-C₆H₄-), (méth)acrylate (CH₂=C(H/CH₃)-CO-O-), (méth)acrylamide (CH₂=C(H/CH₃)-CO-NH- ou CH₂=C(H/CH₃)-CO-NR- où R représente un groupe alkyle en C₁-C₄), de préférence un groupe (méth)acrylate ou (méth)acrylamide, et surtout, un groupe acrylate (CH₂=CH-CO-O-) ou acrylamide (CH₂=CH-CO-NH-),
- HG représente un groupe carboxyle (-COOH),
- Y ne représente rien ou représente un groupe aliphatique à chaîne linéaire comportant de 2 à 10 atomes de carbone, un groupe aliphatique à chaîne ramifiée comportant de 3 à 12 atomes de carbone, ou un groupe cycloaliphatique, bicyclique ou tricyclique ou aromatique comportant de 6 à 15 atomes de carbone, ou une combinaison de tels groupes,
- Z ne représente rien ou représente un groupe aliphatique à chaîne linéaire comportant de 2 à 10 atomes de carbone, un groupe aliphatique à chaîne ramifiée comportant de 3 à 12 atomes de carbone, ou un groupe cycloaliphatique, bicyclique ou tricyclique ou aromatique comportant de 6 à 15 atomes de carbone, une combinaison de tels groupes, ou un groupe de type oligoéthylène-glycol (soit -(CH₂-CH₂-O)ₙ- où l'indice n vaut de 1 à 5) ou oligo-propylèneglycol (soit -(CH(CH₃)-CH₂-O)ₙ- où l'indice n vaut de 1 à 5),
- l'indice a vaut 1, 2 ou 3,
- et l'indice b vaut 1.

3. Barbotine conforme à la revendication 2, dans laquelle les symboles de la formule I ont les significations suivantes :
- X ne représente rien ou représente un groupe de valence (a+b), sans substituant, de type aliphatique à chaîne linéaire ou ramifiée comportant de 3 à 15 et de préférence de 3 à 8 atomes de carbone, qui peut comporter un ou deux groupe(s) de type éther,
- PG représente un groupe acrylate ou acrylamide,
- HG représente un groupe carboxyle (-COOH),
- Y représente un groupe divalent, aliphatique à chaîne linéaire comportant de 2 à 8 atomes de carbone, ou aliphatique à chaîne ramifiée comportant de 3 à 8 atomes de carbone, lequel groupe peut comporter un groupe de type ester,
- Z représente un groupe divalent, aliphatique à chaîne linéaire comportant de 2 à 10 atomes de carbone ou aliphatique à chaîne ramifiée comportant de 3 à 10 atomes de carbone, lequel groupe peut comporter un ou plusieurs groupe(s) de type éther et en comporte de préférence de 0 à 3,
- l'indice a vaut 1, 2 ou 3,
- et l'indice b vaut 1.

4. Barbotine conforme à l'une des revendications 1 à 3, qui contient en outre
D) de 0,001 à 1,0 % en poids d'inhibiteur,
pourcentage rapporté à la masse totale de la barbotine.

5. Barbotine conforme à l'une des revendications 1 à 4, qui contient en outre
- de 0 à 20 % en poids d'accélérateur de déliaison (E),
- de 0 à 20 % en poids de plastifiant,
- et de 0 à 70 % en poids de solvant,
étant entendu que chaque pourcentage est rapporté à la masse totale des composants polymérisables par voie radicalaire.

6. Barbotine conforme à l'une des revendications 1 à 5, qui contient en outre
F) de 0,00001 à 2,0 % en poids de composants colorants, pourcentage rapporté à la masse totale des composants (C).

7. Barbotine conforme à l'une des revendications 1 à 6, dans laquelle les particules des composants (C) présentent une surface qui a été modifiée
- avec un acide carboxylique à chaîne linéaire ou ramifiée, en particulier avec de l'acide formique, acétique, propionique, octanoïque, isobutyrique, isovalérique, ou pivalique ;
- avec un ester phosphate acide, en particulier avec du phosphate de diméthyle, de diéthyle, de dipropyle, de dibutyle, de dipentyle, de dihexyle, de dioctyle, ou de di(2-éthyl-hexyle) ;
- avec un acide phosphonique, en particulier avec de l'acide méthane-phosphonique, éthanephosphonique, propanephosphonique, butane-phosphonique, hexanephosphonique, octanephosphonique, ou benzènephosphonique ;
- ou avec un silane, en particulier avec du propyl-triméthoxy-silane, du phényl-triméthoxy-silane, de l'hexyl-triméthoxy-silane, de l'octyl-triméthoxy-silane, du triméthyl-chloro-silane, du triméthyl-bromo-silane, du triméthyl-méthoxy-silane, ou de l'hexaméthyl-disilazane,
étant entendu que l'agent de modification de surface ne comporte pas de groupes polymérisables par voie radicalaire.

8. Barbotine conforme à l'une des revendications 1 à 6, dans laquelle les particules des composants (C) ont une taille située dans l'intervalle allant de 20 nm à 50 µm.

9. Barbotine conforme à l'une des revendications 5 à 8, qui contient, en tant qu'accélérateur de déliaison (E),
- un agent de régulation des chaînes, en particulier un mercaptan, un disulfure ou un photoamorceur-transporteur-terminateur (« photo-iniferter »), de préférence du lauryl-mercaptan, un dithiouréthane-disulfure, du disulfure de tétraméthyl-thiurame ou du disulfure de xanthogénate d'isopropyle ;
- et/ou un comonomère, qui comporte un ou plusieurs groupe(s) thermolabile(s), en particulier un ou plusieurs groupe(s) de type peroxyde, azo ou uréthane ;
- et/ou un comonomère dont la température plafond vaut de -10 à 150 °C, en particulier de l'a-méthyl-styrène, du polytétrahydro-furane (PTHF) ou un composé téléchélique comportant des groupes polymérisables par voie radicalaire, en particulier un composé téléchélique di(méth)acrylate de PTHF.

10. Barbotine conforme à l'une des revendications 6 à 9, qui contient, en tant que composant colorant (F), un composé de métal de transition, en particulier un acétyl-acétonate et/ou un carboxylate de l'un des éléments suivants : fer, cérium, praséodyme, terbium, lanthane, tungstène, osmium, terbium et manganèse, en particulier de l'acétate de fer-(III) ou de l'acétyl-acétonate de fer-(III), de l'acétate de manganèse-(III) ou de l'acétyl-acétonate de manganèse-(III), de l'acétate de praséodyme-(III) ou de l'acétyl-acétonate de praséodyme-(III), ou de l'acétate de terbium-(III) ou de l'acétyl-acétonate de terbium-(III).

11. Barbotine conforme à l'une des revendications 1 à 10, qui contient en outre au moins un autre composant choisi dans l'ensemble formé par les agents dispersants, les agents anti-mousse et les agents anti-peau.

12. Barbotine conforme à l'une des revendications 1 à 11, qui contient au plus 40 % en poids de monomères polyfonctionnels, pourcentage rapporté à la masse totale des composants polymérisables par voie radicalaire.

13. Utilisation d'une barbotine conforme à l'une des revendications 1 à 12 pour la fabrication de pièces façonnées en céramique ou en vitrocéramique.

14. Utilisation conforme à la revendication 13, dans laquelle la pièce façonnée en céramique est une pièce de restauration dentaire, comme par exemple une incrustation « in-lay » ou « on-lay », une facette « veneer », une couronne, un bridge ou une armature.

15. Procédé de fabrication d'une pièce façonnée en céramique ou en vitrocéramique, dans lequel
a) on prépare un corps cru, en faisant durcir une barbotine conforme à l'une des revendications 1 à 10, par apport local d'énergie de rayonnement, tout en donnant au corps cru une forme géométrique,
b) on soumet ensuite le corps cru à un traitement thermique pour en chasser le liant (déliaison), afin d'obtenir un corps blanc,
c) et l'on fritte ce corps blanc.
